# EUROPEAN PATENT APPLICATION

(11) **EP 2 857 020 A1**
(43) Date of publication of application: **08.04.2015**
(21) Application number: 13799969.4
(22) Date of filing: 14.05.2013
(51) Int. Cl.: A61K 31/55, A61K 9/70, A61K 47/12, A61K 47/14, A61K 47/32, A61K 47/34

(54) **MIRTAZAPINE-CONTAINING TRANSDERMALLY-ABSORBABLE SKIN-ADHESIVE PREPARATION**

(30) Priority: 05.06.2012 JP 2012128306
(71) Applicant: Yutoku Pharmaceutical Industries Co., Ltd., Kashima-shi, Saga 849-1393 (JP)
(72) Inventor: YOSHITAKE, Makoto, Kashima-shi Saga 849-1393 (JP); NISHIMURA, Kenta, Kashima-shi Saga 849-1393 (JP)
(74) Representative: Blodig, Wolfgang
(86) International application number: PCT/JP2013/063415
(87) International publication number: WO 2013/183407

(57) **Abstract**

The purpose is to provide a mirtazapine-containing transdermal patch capable of suppressing deposition of a crystalline component derived from mirtazapine, deterioration in sense of use, and deterioration in adhesiveness to the skin. The transdermal patch contains a support, a drug-containing layer and a release liner, and the drug-containing layer contains mirtazapine and an organic acid.

## Description

### Technical Field

The present invention relates to a transdermal patch containing a support, a drug-containing layer and a release liner, in which the drug-containing layer contains, as the active ingredient, mirtazapine and an organic acid.

### Background Art

Mirtazapine is a type of a noradrenergic and specific serotonergic antidepressant (NaSSA), and is an antidepressant that exhibits an antagonistic effect to central presynaptic α2 adrenalin autoreceptor and heteroreceptor and has an effect of enhancing the neurotransmission of both central serotonin (5-HT) and noradrenalin (NA). NaSSA has a functional mechanism different from that of other antidepressants such as a selective serotonin reuptake inhibitor (SSRI), for example, fluvoxamine or the like and a serotonin/norepinephrine reuptake inhibitor (SNRI), for example, milnacipran or the like, and is considered to promote release of 5-HT and NA to thereby exhibit the antidepressant effect on a level equivalent to or higher than that of SSRI and SNRI. Mirtazapine that is the sole commercially available pharmaceutical ingredient as NaSSA is characterized by its ability to not only promote release of 5-HT and NA but also to promote release of dopamine (DA). At present, mirtazapine is used as a therapeutic medicine for depression and anxiety disorder but only in the form of an oral preparation. Further, mirtazapine is under clinical research as a therapeutic medicine for pain, such as fibromyalgia.

As compared with SSRI and others, NaSSA has few side effects such as nausea and vomiting, sexual dysfunction, but may express sedative side effects such as somnolentia, dry mouth, fatigue and the like due to the strong antihistamine effect and muscarinic anticholinergic effect, and is therefore known to be problematic in terms of stabilizing the blood level and sustaining its effect.

In recent years, external preparations containing a centrally-acting pharmaceutical agent such as an antidepressant have been developed. For example, there are disclosed transdermal patches containing SSRI such as fluoxetine, sertraline, fluvoxamine, paroxetine or the like (Patent Document 1, Patent Document 2). Whilst these documents evidence transdermal absorption of SSRI, inhibition of skin irritation by SSRI, and inhibition of side effects owing to rapid blood level increase achieved by transdermal patches containing SSRI. They do not disclose a NaSSA-containing transdermal patch or its composition.

As described above, NaSSA and SSRI differ in the functional mechanism and the side effects thereof. There is no disclosure of the usefulness and the safety of a NaSSA-containing transdermal patch, not to mention the relationship between the blood level and the potency in administration of a NaSSA-containing transdermal patch. Moreover, in the related art, there is no suggestion of an external preparation of a type which enables transdermal absorption of a sufficient amount of NaSSA for expressing the therapeutic effect thereof and its usefulness.

Under the circumstances, the present inventors have made earnest investigations for providing an NaSSA-containing transdermal patch.
Patent Document 1: JP-A 2006-335714
Patent Document 2: JP-A 2007-284378

### Summary of Invention

### Technical Problem

As a result of repeated earnest investigations for providing a NaSSA-containing transdermal patch capable of inhibiting the expression of side effects that are problematic in oral administration, and capable of enhancing the stability of NaSSA, the inventors have developed a novel transdermal patch containing mirtazapine and antioxidant. However, it has been found that on increasing the content of mirtazapine for enhancing the transdermal amount thereof, a crystalline component derived from mirtazapine may be deposited on the surface of an adhesive layer (i.e., the drug-containing layer) after the production of the transdermal patch, and the particle diameter of the crystalline component may be increased with the lapse of time. The deposition of the crystalline component referred herein means the case where particles having a particle diameter exceeding 200 µm per one particle are present in the crystals deposited on the surface of the adhesive layer with the lapse of time, and does not include the case where no crystal is visually confirmed, and the case where all the particles have a particle diameter of 200 µm or less per one particle.

There has been demands for prevention of the formation of the crystalline component since the presence of the crystalline component may create an impression of deteriorated quality on the patient, and the increase in the particle diameter of the crystalline component may cause problems including deterioration in sense of use of the transdermal patch, and the deterioration in adhesiveness thereof to the skin. In the mirtazapine-containing transdermal patch, the deposition of the crystalline component derived from mirtazapine and the increase in the particle diameter of the crystalline component with the lapse of time are problems that have not been known but are found for the first time through the investigations made by the inventors.

### Solution to Problem

As a result of further earnest investigations for solving the problems, the inventors have developed a novel transdermal patch containing mirtazapine and an organic acid. Specifically, it has been found that the problems may be solved by a transdermal patch containing a support, a drug-containing layer and a release liner, which contains a therapeutically or preventively effective amount of mirtazapine, and an organic acid, and thus the present invention has been completed.

As a result of the investigations, in particular, the inventors confirmed for the first time that in a transdermal patch containing mirtazapine, a solubilizer that has been ordinarily used, such as crotamiton, N-methyl-2-pyrrolidone or oleyl alcohol, is insufficient for suppressing the deposition of crystals of mirtazapine, and an organic acid is effective for the suppression, with a particular organic acid being significantly effective.

Therefore, the inventors have found that an organic acid contained in the composition may suppress the deposition of the crystalline component derived from mirtazapine on the surface of the adhesive layer with the lapse of time and may provide a preparation having quality and skin adhesiveness that are sufficient for the use.

### Effects of Invention

The present invention relates to a novel transdermal patch containing mirtazapine, which is capable of preventing the expression of side effects that are problematic in oral preparations, by being adhered to the skin of the patient. The transdermal patch of the invention contains mirtazapine and an organic acid in the drug-containing layer. In the investigations on the patch, it has been found that the deposition of the crystalline component derived from mirtazapine in the drug-containing layer may be suppressed, and thereby the deterioration over time in the quality, the sense of use and/or the skin adhesion force of the mirtazapine-containing transdermal patch may be prevented. As a result, the preparation may be stored for a prolonged period of time, and the pharmacological effects of mirtazapine may be utilized effectively and sustainably. Another advantage of the transdermal patch of the invention is that the blood level of mirtazapine can be controlled and therefore the active ingredient can be readily administered in an amount necessary for expression of the pharmacological potency thereof but smaller than the level thereof to express side effects. Even when any undesirable effects are expressed, administration can be immediately stopped by just removing the patch. Accordingly, the transdermal patch of the invention is an excellent preparation in terms of safety as compared with oral preparations, etc. Further, like oral antidepressants, the transdermal patch of the invention can be used favorably as differentiated from any other transdermal patches containing an antidepressant that has a different functional mechanism such as SSRI or the like, depending on the depressive symptoms of the patient; and consequently, the transdermal patch of the invention broadens the patients' latitude in selecting pharmaceutical preparations in treatment for depression, and is therefore therapeutically useful.

### Detailed Description of Embodiments

The transdermal patch of the invention contains a drug-containing layer formed on the support thereof. Until use, a release liner is kept provided on the drug-containing layer for the purpose of protecting the drug-containing layer.

The drug-containing layer contains mirtazapine as the active ingredient therein. Mirtazapine is a drug of which the past record of actual use for treatment for depression and anxiety disorder as oral preparations has been verified and of which clinical studies as therapeutic medicines for pain, such as fibromyalgia are being promoted.

The transdermal patch of the invention contains a therapeutically effective amount of mirtazapine. For long-term continuous transdermal administration of the active ingredient to a patient suffering from depression, anxiety disorder or fibromyalgia, in an amount effective for treatment for depression, anxiety disorder, fibromyalgia or the like and in an amount expressing few side effects, it is important to incorporate a predetermined amount of the active ingredient in the drug-containing layer.

In the description of the present invention, the transdermal patch means an adhesive patch for medical treatment, which, when stuck to skin, releases a therapeutically effective amount of the active ingredient to pass through the skin to reach the blood.

The content of the active ingredient in the transdermal patch of the invention is preferably from 3 to 30% by mass, and more preferably from 5 to 25% by mass relative to the drug-containing layer. When the content is less than 5% by mass, the area of the preparation needs to be increased to provide the sufficient transdermal amount. When the content exceeds 25% by mass, it may be difficult to keep good physical properties of the patch in some cases.

The active ingredient to be contained in the transdermal patch of the invention may be in the form of a pharmaceutically-acceptable salt thereof. Examples of the pharmaceutically-acceptable salt of mirtazapine include acid addition salts with inorganic acids or organic acids, examples of which include hydrochloride, hydrobromide, nitrate, phosphate, sulfate, acetate, ascorbate, benzoate, cinnamate, citrate, formate, fumarate, glutamate, lactate, maleate, malate, malonate, mandelate, methanesulfonate (mesilate), phthalate, salicylate, stearate, succinate, tartrate, propionate, butyrate, pamonate, p-toluenesulfonate (tosylate), but the pharmaceutically-acceptable salt is not limited thereto. In the invention, a free form of mirtazapine is preferably used.

The drug-containing layer of the transdermal patch of the invention contains an organic acid for suppressing the deposition of the crystalline component derived from mirtazapine and the increase in the particle diameter thereof with the lapse of time. The organic acid is preferably an organic acid having from 2 to 18 carbon atoms, and a linear or branched aliphatic acid having from 8 to 18 carbon atoms and a hydroxycarboxylic acid having from 1 to 6 carbon atoms are preferred, one or more kinds of which may be used. Examples of the linear or branched aliphatic acid having from 8 to 18 carbon atoms include caprylic acid, azelaic acid, capric acid, undecenoic acid, lauric acid, myristic acid, brassylic acid, palmitic acid, isostearic acid, stearic acid and oleic acid. Examples of the hydroxycarboxylic acid having from 1 to 6 carbon atoms include lactic acid, citric acid, glycolic acid, tartaric acid and mevalonic acid. Among these, at least one selected from glycolic acid, citric acid, caprylic acid, azelaic acid, capric acid, undecenoic acid, lauric acid, myristic acid, isostearic acid, stearic acid, oleic acid and lactic acid is preferred since they are excellent in crystal deposition suppressing effect.

The content of the organic acid in the transdermal patch of the invention is preferably from 2 to 30% by mass, and more preferably from 3 to 24% by mass relative to the drug-containing layer. When the content is less than 3% by mass, the effect of suppressing the deposition of the crystalline component derived from mirtazapine with the lapse of time may not be observed in some cases. When the content exceeds 24% by mass, it may be difficult to keep good physical properties of the patch in some cases.

The amount of the organic acid in the transdermal patch of the invention is preferably from 0.3 to 11.0 times by mole, and more preferably from 0.5 to 9.0 times by mole, the content of mirtazapine. When the amount is less than 0.5 times by mole, the effect of suppressing the deposition of the crystalline component derived from mirtazapine may not be observed in some cases. When the amount exceeds 9.0 times by mole, it may be difficult to keep good physical properties of the patch in some cases.

The crystalline component derived from mirtazapine present in the drug-containing layer of the transdermal patch of the invention may have a size of 200 µm or less, and preferably 100 µm or less. The size of the crystalline component present in the drug-containing layer that exceeds 200 µm is unfavorable since the presence of the crystalline component may be confirmed visually, which may be determined as deteriorated quality on using by the patient in some cases, and problems including deteriorated skin adhesiveness due to the crystal deposition may occur in some cases.

The transdermal patch of the invention contains a support, a drug-containing layer and a release liner. Further, if desired, a release control film may be added to the skin-application side of the drug-containing layer for controlling the transdermal absorption of the active ingredient through the skin, and an adhesive layer may be added thereto for applying the patch to skin. Moreover, a reservoir-type patch may also be employed.

In the transdermal patch of the invention, the drug-containing layer is preferably a matrix-type adhesive layer that contains the active ingredient and, as the base ingredient thereof, an adhesive ingredient. In case where the patch of the invention is a matrix-type patch that contains such a matrix-type adhesive layer, the preparation for the patch can be easily manufactured and the patch does not require any additional layer such as an adhesive layer or the like, and consequently, the production cost for the patch can be reduced.

The adhesive ingredient to be contained in the drug-containing layer of the transdermal patch of the invention is preferably a nonaqueous adhesive ingredient, including a rubber-type adhesive ingredient, an acrylic polymer and a silicone polymer.

The rubber-type adhesive ingredient includes one or more selected from styrene-isoprene-styrene block copolymer, styrene-butadiene-styrene block copolymer, styrene-butadiene rubber, polyisobutylene, polybutene, polyisoprene, butyl rubber, natural rubber and isoprene rubber; and any of these may be used here.

The acrylic polymer includes, though not defined thereto, polymers or copolymers containing, as the monomer unit thereof, at least one (meth)acrylate of typically 2-ethylhexyl acrylate, methyl acrylate, butyl acrylate, 2-hydroxyethyl acrylate, 2-ethylhexyl methacrylate or the like. For example, herein usable are adhesives of acrylic polymers and the like that contain acrylic acid/octyl acrylate copolymer, 2-ethylhexyl acrylate/N-vinyl-2-pyrrolidone/1,6-hexaneglycol dimethacrylate copolymer, 2-ethylhexyl acrylate/vinyl acetate copolymer, 2-ethylhexyl acrylate/vinyl acetate/acrylic acid copolymer, 2-ethylhexyl acrylate/2-ethylhexyl methacrylate/dodecyl methacrylate copolymer, methyl acrylate/2-ethylhexyl acrylate copolymer resin emulsion, or acrylic resin alkanolamine liquid; and for example, usable are commercially-available DURO-TAK™ acrylic adhesive series (by Henkel Japan), GELVA™ acrylic adhesive series (by Monsanto), SK-DYNE MATRIDERM (by Soken Chemical), EUDRAGIT™ series (by Higuchi Shokai), etc.

The silicone polymer includes polysiloxane derivatives (for example, silicone polymers such as polydimethylsiloxane, amine-resistant polydimethylsiloxane, etc.), etc.

The amount of the adhesive ingredient to be incorporated in the drug-containing layer is from 20 to 91% by mass, preferably from 24 to 79% by mass relative to the drug-containing layer, in consideration of formation of the drug-containing layer and of sufficient drug releasability.

As the adhesive ingredient to be contained in the drug-containing layer of the transdermal patch of the invention, one kind or a combination of two or more kinds selected from the the above-mentioned rubber-type adhesive ingredients, acrylic polymers and silicone polymers may be contained. The drug-containing layer of the transdermal patch of the invention is preferably a nonaqueous system containing substantially no water.

If desired, the transdermal patch of the invention may contain any other additional ingredients such as a plasticizer, a crosslinking agent, a colorant, a UV absorbent, a tackifier, an absorption promoter and an antioxidant.

Examples of the plasticizer include petroleum oils such as paraffinic process oil, naphthenic process oil, aromatic process oil, etc.; liquid fatty acid esters such as isopropyl myristate, hexyl laurate, diethyl sebacate, diisopropyl sebacate, isopropyl linoleate, etc.; vegetable oils such as olive oil, camellia oil, castor oil, tall oil, peanut oil, etc.; glycerin, chlorobutanol, vinyl acetate resin, dimethylpolysiloxane-silicon dioxide mixture, D-sorbitol, middle-chain fatty acid triglyceride, triacetin, 2-pyrrolidone, phytosterol, propylene glycol, polyethylene glycol, Polysorbate 80™, glycerin monostearate, etc.

Examples of the crosslinking agent include thermosetting resins such as amino resin, phenolic resin, epoxy resin, alkyd resin, unsaturated polyester, etc.; isocyanate compounds, organic crosslinking agents; inorganic crosslinking agents such as metals or metal compounds, etc.

Examples of the colorant include indigocarmine, yellow iron oxide, yellow iron sesquioxide, carbon black, caramel, Photopigment 201, dwarf bamboo extract, black iron oxide, dragon's blood palm, zinc oxide, titanium oxide, iron sesquioxide, amaranth, sodium hydroxide, talc, sodium copper chlorophyllin, hull-less barley green leaf extract powder, d-borneol, octyldodecyl myristate, methylrosanilinium chloride, methylene blue, ammonium manganese phosphate, rose oil, etc.

Examples of the UV absorbent include amino acid compounds such as urocanic acid, etc.; benzophenone compounds such as 2,4-dihydroxybenzophenone, 2-hydroxy-4-n-octoxybenzophenone, etc.; cinnamic acid derivatives such as cinoxate, p-methoxycinnamic acid diethanolamine, etc.; cyanoacrylate derivatives such as 2-ethylhexyl-2-cyano-3,3'-diphenyl acrylate, etc.; p-aminobenzoic acid derivatives such as ethyl p-aminobenzoate, propyl p-aminobenzoate, etc.; anthranilic acid derivatives such as menthyl anthranilate, etc.; salicylic acid derivatives such as phenyl salicylate, p-octylphenyl salicylate, etc.; coumarin derivatives such as 7-ethylamino-4-methylcoumarin, 7,8-dihydroxycoumarin, etc.

Examples of the tackifier include rosin derivatives such as rosin, rosin glycerin ester, hydrogenated rosin, hydrogenated rosin glycerin ester, etc.; aliphatic saturated hydrocarbon resins, aliphatic hydrocarbon resins, terpene resins, resin maleate, carnauba wax, sodium carmellose, xanthane gum, chitosan, glycerin, magnesium aluminium silicate, light anhydrous silicic acid, benzyl acetate, talc, hydroxyethyl cellulose, hydroxypropyl cellulose, hypromellose, polyacrylic acid, sodium polyacrylate, partially-neutralized polyacrylic acid, polyvinyl alcohol, etc. Among these, a rosin derivative is preferably contained since it is excellent in the crystal deposition suppressing effect.

The amount of the tackifier contained in the drug-containing layer may be from 8 to 46% by mass, and preferably from 30 to 46% by mass relative to the drug-containing layer, in consideration of the sufficient skin adhesiveness.

Examples of the antioxidant include phenolic antioxidants, ascorbic acid and its ester derivatives, sodium hydrogensulfite, sodium pyrosulfite, sodium edetate, citric acid, potassium dichloroisocyanurate, soybean lecithin, thymol, tocopherol and its ester derivatives, 1,3-butylene glycol, benzotriazole, monothioglycerol, etc. Of those, preferred are ascorbic acid and its esters, or phenolic antioxidants.

As ascorbic acid and an ester thereof, there are mentioned L-ascorbic acid, L-ascorbyl palmitate, L-ascorbyl stearate, L-ascorbic acid 2-glucoside, sodium L-ascorbate, calcium L-ascorbate, magnesium L-ascorbyl phosphate, isoascorbic acid, sodium isoascorbate, etc. Above all, preferred are L-ascorbyl palmitate and isoascorbic acid.

The phenolic antioxidant includes dibutylhydroxytoluene, butylhydroxyanisole, propyl gallate, octyl gallate, lauryl gallate, nordihydroguairaretic acid, trihydroxybutyrophenone, tert-butylhydroquinone, 4-hydroxymethyl-2,6-di-tert-butylphenol, etc. Above all, preferred is dibutylhydroxytoluene.

The transdermal absorption promoter may be any compound that has heretofore been recognized to exhibit an absorption-promoting effect in transdermal administration, and includes, for example, alkanolamines such as diisopropanolamine, triisopropanolamine, etc.; aliphatic acid esters such as isopropyl myristate, octyldodecyl myristate, oleic acid glycerol monoester, hexadecyl isostearate, etc.; alcohols or their esters or ethers such as oleyl alcohol, propylene glycol, polyethylene glycol monooleate, etc.; sorbitan esters or ethers such as sorbitan monolaurate, sorbitan monooleate, etc.; phenol ethers such as polyoxyethylene nonylphenyl ether, polyoxyethylene octylphenyl ether, etc.; castor oil or hardened castor oil; ionic surfactants such as oleoyl sarcosine, lauryldimethylaminoacetate betaine, sodium laurylsulfate, etc.; nonionic surfactants such as polyoxyethylene oleyl ether, polyoxyethylene lauryl ether, dimethyllaurylamine oxide, etc.; alkylmethyl sulfoxides such as dimethyl sulfoxide, decylmethyl sulfoxide, etc.; pyrrolidones such as 2-pyrrolidone, 1-methyl-2-pyrrolidone, etc.; azacycloalkanes such as 1-dodecylazacycloheptan-2-one, 1-geranylazacycloheptan-2-one, etc.; terpenes such as menthol, camphor, limonene, etc. Of those, preferred are myristates such as isopropyl myristate, octyldodecyl myristate, etc.; sebacates such as diisopropyl sebacate, etc.; menthol, polyoxyethylene oleyl ether or Polysorbate 80™.

The amount of the transdermal absorption promoter, if any, in the transdermal patch of the invention may be from 0.1 to 15% by mass, preferably from 1 to 10% by mass relative to the drug-containing layer. The amount of less than 0.1% by mass is unfavorable, since no absorption-promoting effect may be observed. On the other hand, the amount of more than 15% by mass is also unfavorable, since there may occur skin irritation derived from the transdermal absorption promoter and the physical properties of the preparation may deteriorate and cause stickiness.

As the support of the transdermal patch of the invention, a drug-impervious elastic or nonelastic support may be used. The support of the type includes, for example, synthetic resin films or sheets or their laminates of polyethylene, polypropylene, polybutadiene, ethylene-vinyl acetate copolymer, polyvinyl chloride, polyester (polyethylene terephthalate, etc.), nylon, polyurethane, etc.; porous substances, foams, papers, woven fabrics, nonwoven fabrics, etc.

As the release liner of the transdermal patch of the invention, a drug-impervious release liner may be used. The release liner includes, for example, films formed of a polymer material such as polyethylene, polypropylene, polyester or the like; those produced through vapor deposition of aluminium on a film; those produced by coating paper with silicone oil or the like, etc. Above all, preferred are polyester films since the active ingredient does not permeate through them and from the viewpoint of the workability and the low cost thereof; and more preferred are polyethylene terephthalate (PET) films. Further, as the release liner, also usable is a laminate film produced through lamination of multiple materials, etc.

There is no specific limitation on the transdermal patch of the invention and it can be produced according to any known production method. Preferred known production methods for the transdermal patch of the invention include a method that comprises, for example, dissolving an active ingredient and an adhesive and optionally a transdermal absorption promoter in an organic solvent of ethyl acetate, hexane, toluene or a mixed solvent thereof, then spreading the dissolved matter onto a release liner or a support, evaporating away the solvent from the dissolved matter to form a drug-containing layer, and thereafter sticking a support or a release liner thereto to give a transdermal patch; a method that comprises melting an active ingredient and an adhesive and optionally a transdermal absorption promoter under heat, then spreading the resulting melt onto a release liner or a support to form a drug-containing layer thereon, and thereafter sticking a support or a release liner thereto to give a transdermal patch, etc.

### Examples

The present invention is described in more detail with reference to the following Examples, not being limited thereto.

### Example 1

According to the blend ratio shown in Table 1, styrene-isoprene-styrene block copolymer, polybutene, hydrogenated rosin glycerin ester, alicyclic saturated hydrocarbon resin, capric acid, dibutylhydroxytoluene and ascorbyl palmitate were stirred and mixed in toluene, and then mirtazapine was added thereto, and stirred and mixed to give a uniform dissolved matter. Next, using a doctor knife coater, the dissolved matter was spread on a release film (silicone-processed PET film, Fujimori Industry) to have a thickness of 100 µm after solvent removal, then the solvent was evaporated away to form a drug-containing layer, and thereafter a support was stuck thereto. Subsequently, the assembly was cut into 10 cm² to provide a transdermal patch.

### Example 2

A transdermal patch was produced in the same manner as in Example 1 and according to the blend ratio as in Table 1, except that oleic acid was used in place of capric acid.

### Example 3

A transdermal patch was produced in the same manner as in Example 1 and according to the blend ratio as in Table 1, except that caprylic acid was used in place of capric acid.

### Example 4

A transdermal patch was produced in the same manner as in Example 1 and according to the blend ratio as in Table 1, except that isostearic acid was used in place of capric acid.

### Example 5

A transdermal patch was produced in the same manner as in Example 1 and according to the blend ratio as in Table 1, except that stearic acid was used in place of capric acid.

### Example 6

A transdermal patch was produced in the same manner as in Example 1 and according to the blend ratio as in Table 1, except that undecenoic acid was used in place of capric acid.

### Example 7

A transdermal patch was produced in the same manner as in Example 1 and according to the blend ratio as in Table 1, except that lactic acid was used in place of capric acid.

### Example 8

A transdermal patch was produced in the same manner as in Example 1 and according to the blend ratio as in Table 1, except that lauric acid was used in place of capric acid.

### Example 9

A transdermal patch was produced in the same manner as in Example 1 and according to the blend ratio as in Table 1, except that myristic acid was used in place of capric acid.

### Example 10

A transdermal patch was produced in the same manner as in Example 1 and according to the blend ratio as in Table 1, except that azelaic acid was used in place of capric acid.

### Example 11

A transdermal patch was produced in the same manner as in Example 1 and according to the blend ratio as in Table 1, except that glycolic acid was used in place of capric acid.

### Example 12

A transdermal patch was produced in the same manner as in Example 1 and according to the blend ratio as in Table 1, except that citric acid was used in place of capric acid.

### Example 13

A transdermal patch was produced in the same manner as in Example 7 and according to the blend ratio as in Table 1.

### Example 14

A transdermal patch was produced in the same manner as in Example 7 and according to the blend ratio as in Table 1.

### Example 15

A transdermal patch was produced in the same manner as in Example 1 and according to the blend ratio as in Table 1.

### Example 16

A transdermal patch was produced in the same manner as in Example 1 and according to the blend ratio as in Table 1.

### Example 17

A transdermal patch was produced in the same manner as in Example 1 and according to the blend ratio as in Table 1, except that the alicyclic saturated hydrocarbon resin was not mixed.

### Example 18

A transdermal patch was produced in the same manner as in Example 1 and according to the blend ratio as in Table 1, except that the hydrogenated rosin glycerin ester was not mixed.

### Example 19

A transdermal patch was produced in the same manner as in Example 1 and according to the blend ratio as in Table 2.

### Example 20

A transdermal patch was produced in the same manner as in Example 1 and according to the blend ratio as in Table 2.

### Example 21

A transdermal patch was produced in the same manner as in Example 17 and according to the blend ratio as in Table 2.

### Example 22

A transdermal patch was produced in the same manner as in Example 17 and according to the blend ratio as in Table 2.

### Example 23

A transdermal patch was produced in the same manner as in Example 1 and according to the blend ratio as in Table 2.

### Example 24

A transdermal patch was produced in the same manner as in Example 2 and according to the blend ratio as in Table 2.

### Example 25

A transdermal patch was produced in the same manner as in Example 7 and according to the blend ratio as in Table 2.

### Example 26

According to the blend ratio shown in Table 2, acrylic polymer (DURO-TAK™ 87-4287, obtained from Henkel Technologies Japan), capric acid and dibutylhydroxytoluene were stirred and mixed in toluene and thereafter mirtazapine was added thereto, and stirred and mixed to give a uniform dissolved matter. Next, using a doctor knife coater, the dissolved matter was spread on a release film to have a thickness of 100 µm after solvent removal, then the solvent was evaporated away to form a drug-containing layer, and thereafter a support was stuck thereto. Subsequently, the assembly was cut into 10 cm² to provide a transdermal patch.

### Example 27

A transdermal patch was produced in the same manner as in Example 26 and according to the blend ratio as in Table 2, except that octyldodecyl myristate was mixed.

### Comparative Example 1

A transdermal patch was produced in the same manner as in Example 1 and according to the blend ratio as in Table 2, except that capric acid was not mixed.

### Comparative Example 2

A transdermal patch was produced in the same manner as in Example 27 and according to the blend ratio as in Table 2, except that capric acid was not mixed.

### Comparative Example 3

According to the blend ratio shown in Table 2, styrene-isoprene-styrene block copolymer, polybutene, hydrogenated rosin glycerin ester, alicyclic saturated hydrocarbon resin, crotamiton, dibutylhydroxytoluene and ascorbyl palmitate were stirred and mixed in toluene, and then mirtazapine was added thereto, and stirred and mixed to give a uniform dissolved matter. Next, using a doctor knife coater, the dissolved matter was spread on a release film (silicone-processed PET film, Fujimori Industry) to have a thickness of 100 µm after solvent removal, then the solvent was evaporated away to form a drug-containing layer, and thereafter a support was stuck thereto. Subsequently, the assembly was cut into 10 cm² to provide a transdermal patch.

### Comparative Example 4

A transdermal patch was produced in the same manner as in Comparative Example 3 and according to the blend ratio as in Table 2, except that N-methyl-2-pyrrolidone was used in place of crotamiton.

### Comparative Example 5

A transdermal patch was produced in the same manner as in Comparative Example 3 and according to the blend ratio as in Table 2, except that oleyl alcohol was used in place of crotamiton.

### Comparative Example 6

A transdermal patch was produced in the same manner as in Comparative Example 3 and according to the blend ratio as in Table 2, except that sorbitan sesquioleate was used in place of crotamiton.

### Comparative Example 7

A transdermal patch was produced in the same manner as in Comparative Example 3 and according to the blend ratio as in Table 2, except that menthol was used in place of crotamiton.

### Comparative Example 8

A transdermal patch was produced in the same manner as in Comparative Example 3 and according to the blend ratio as in Table 2, except that polyethylene glycol 400 was used in place of crotamiton.

### Comparative Example 9

A transdermal patch was produced in the same manner as in Comparative Example 3 and according to the blend ratio as in Table 2, except that phosphoric acid was used in place of crotamiton.

**Table 1**

| | Example 1 | Example 2 | Example 3 | Example 4 | Example 5 | Example 6 | Example 7 | Example 8 | Example 9 |
|---|---|---|---|---|---|---|---|---|---|
| Mirtazapine | 8.00 | 8.00 | 8.00 | 8.00 | 8.00 | 8.00 | 8.00 | 8.00 | 8.00 |
| Styrene-isoprene-styrene block copolymer | 34.40 | 34.40 | 34.40 | 34.40 | 34.40 | 34.40 | 34.40 | 34.40 | 34.40 |
| Polybutene | 8.60 | 8.60 | 8.60 | 8.60 | 8.60 | 8.60 | 8.60 | 8.60 | 8.60 |
| Hydrogenated rosin glycerin ester | 14.33 | 14.33 | 14.33 | 14.33 | 14.33 | 14.33 | 14.33 | 14.33 | 14.33 |
| Alicyclic saturated hydrocarbon resin | 28.65 | 28.65 | 28.65 | 28.65 | 28.65 | 28.65 | 28.65 | 28.65 | 28.65 |
| DURO-TAK87-4287 | 0.00 | 0.00 | 0.00 | 0.00 | 0.00 | 0.00 | 0.00 | 0.00 | 0.00 |
| Capric acid | 5.00 | 0.00 | 0.00 | 0.00 | 0.00 | 0.00 | 0.00 | 0.00 | 0.00 |
| Oleic acid | 0.00 | 5.00 | 0.00 | 0.00 | 0.00 | 0.00 | 0.00 | 0.00 | 0.00 |
| Caprylic acid | 0.00 | 0.00 | 5.00 | 0.00 | 0.00 | 0.00 | 0.00 | 0.00 | 0.00 |
| Isostearic acid | 0.00 | 0.00 | 0.00 | 5.00 | 0.00 | 0.00 | 0.00 | 0.00 | 0.00 |
| Stearic acid | 0.00 | 0.00 | 0.00 | 0.00 | 5.00 | 0.00 | 0.00 | 0.00 | 0.00 |
| Undecenoic acid | 0.00 | 0.00 | 0.00 | 0.00 | 0.00 | 5.00 | 0.00 | 0.00 | 0.00 |
| Lactic acid | 0.00 | 0.00 | 0.00 | 0.00 | 0.00 | 0.00 | 5.00 | 0.00 | 0.00 |
| Lauric acid | 0.00 | 0.00 | 0.00 | 0.00 | 0.00 | 0.00 | 0.00 | 5.00 | 0.00 |
| Myristic acid | 0.00 | 0.00 | 0.00 | 0.00 | 0.00 | 0.00 | 0.00 | 0.00 | 5.00 |
| Azelaic acid | 0.00 | 0.00 | 0.00 | 0.00 | 0.00 | 0.00 | 0.00 | 0.00 | 0.00 |
| Glycolic acid | 0.00 | 0.00 | 0.00 | 0.00 | 0.00 | 0.00 | 0.00 | 0.00 | 0.00 |
| Citric acid | 0.00 | 0.00 | 0.00 | 0.00 | 0.00 | 0.00 | 0.00 | 0.00 | 0.00 |
| Crotamiton | 0.00 | 0.00 | 0.00 | 0.00 | 0.00 | 0.00 | 0.00 | 0.00 | 0.00 |
| N-methyl-2-pyrrolidone | 0.00 | 0.00 | 0.00 | 0.00 | 0.00 | 0.00 | 0.00 | 0.00 | 0.00 |
| Oleyl alcohol | 0.00 | 0.00 | 0.00 | 0.00 | 0.00 | 0.00 | 0.00 | 0.00 | 0.00 |
| Sorbitan sesquioleate | 0.00 | 0.00 | 0.00 | 0.00 | 0.00 | 0.00 | 0.00 | 0.00 | 0.00 |
| Menthol | 0.00 | 0.00 | 0.00 | 0.00 | 0.00 | 0.00 | 0.00 | 0.00 | 0.00 |
| Polyethylene glycol 400 | 0.00 | 0.00 | 0.00 | 0.00 | 0.00 | 0.00 | 0.00 | 0.00 | 0.00 |
| Phosphoric acid | 0.00 | 0.00 | 0.00 | 0.00 | 0.00 | 0.00 | 0.00 | 0.00 | 0.00 |
| Octyldodecyl myristate | 0.00 | 0.00 | 0.00 | 0.00 | 0.00 | 0.00 | 0.00 | 0.00 | 0.00 |
| Dibutylhydroxytoluene | 1.00 | 1.00 | 1.00 | 1.00 | 1.00 | 1.00 | 1.00 | 1.00 | 1.00 |
| Ascorbyl palmitate | 0.02 | 0.02 | 0.02 | 0.02 | 0.02 | 0.02 | 0.02 | 0.02 | 0.02 |
| Organic acid/mirtazapine (molar ratio) | 0.96 | 0.59 | 1.15 | 0.58 | 0.58 | 0.90 | 1.84 | 0.83 | 0.73 |

| | Example 10 | Example 11 | Example 12 | Example 13 | Example 14 | Example 15 | Example 16 | Example 17 | Example 18 |
|---|---|---|---|---|---|---|---|---|---|
| Mirtazapine | 8.00 | 8.00 | 8.00 | 8.00 | 8.00 | 3.00 | 5.00 | 5.00 | 8.00 |
| Styrene-isoprene-styrene block copolymer | 34.40 | 34.40 | 34.40 | 35.19 | 26.62 | 37.19 | 36.39 | 36.39 | 34.40 |
| Polybutene | 8.60 | 8.60 | 8.60 | 8.80 | 6.65 | 9.30 | 9.10 | 9.10 | 8.60 |
| Hydrogenated rosin glycerin ester | 14.33 | 14.33 | 14.33 | 14.67 | 11.09 | 15.50 | 15.17 | 45.49 | 0.00 |
| Alicyclic saturated hydrocarbon resin | 28.65 | 28.65 | 28.65 | 29.32 | 22.18 | 30.99 | 30.32 | 0.00 | 42.98 |
| DURO-TAK87-4287 | 0.00 | 0.00 | 0.00 | 0.00 | 0.00 | 0.00 | 0.00 | 0.00 | 0.00 |
| Capric acid | 0.00 | 0.00 | 0.00 | 0.00 | 0.00 | 3.00 | 3.00 | 3.00 | 5.00 |
| Oleic acid | 0.00 | 0.00 | 0.00 | 0.00 | 0.00 | 0.00 | 0.00 | 0.00 | 0.00 |
| Caprylic acid | 0.00 | 0.00 | 0.00 | 0.00 | 0.00 | 0.00 | 0.00 | 0.00 | 0.00 |
| Isostearic acid | 0.00 | 0.00 | 0.00 | 0.00 | 0.00 | 0.00 | 0.00 | 0.00 | 0.00 |
| Stearic acid | 0.00 | 0.00 | 0.00 | 0.00 | 0.00 | 0.00 | 0.00 | 0.00 | 0.00 |
| Undecenoic acid | 0.00 | 0.00 | 0.00 | 0.00 | 0.00 | 0.00 | 0.00 | 0.00 | 0.00 |
| Lactic acid | 0.00 | 0.00 | 0.00 | 3.00 | 24.44 | 0.00 | 0.00 | 0.00 | 0.00 |
| Lauric acid | 0.00 | 0.00 | 0.00 | 0.00 | 0.00 | 0.00 | 0.00 | 0.00 | 0.00 |
| Myristic acid | 0.00 | 0.00 | 0.00 | 0.00 | 0.00 | 0.00 | 0.00 | 0.00 | 0.00 |
| Azelaic acid | 5.00 | 0.00 | 0.00 | 0.00 | 0.00 | 0.00 | 0.00 | 0.00 | 0.00 |
| Glycolic acid | 0.00 | 5.00 | 0.00 | 0.00 | 0.00 | 0.00 | 0.00 | 0.00 | 0.00 |
| Citric acid | 0.00 | 0.00 | 5.00 | 0.00 | 0.00 | 0.00 | 0.00 | 0.00 | 0.00 |
| Crotamiton | 0.00 | 0.00 | 0.00 | 0.00 | 0.00 | 0.00 | 0.00 | 0.00 | 0.00 |
| N-methyl-2-pyrrolidone | 0.00 | 0.00 | 0.00 | 0.00 | 0.00 | 0.00 | 0.00 | 0.00 | 0.00 |
| Oleyl alcohol | 0.00 | 0.00 | 0.00 | 0.00 | 0.00 | 0.00 | 0.00 | 0.00 | 0.00 |
| Sorbitan sesquioleate | 0.00 | 0.00 | 0.00 | 0.00 | 0.00 | 0.00 | 0.00 | 0.00 | 0.00 |
| Menthol | 0.00 | 0.00 | 0.00 | 0.00 | 0.00 | 0.00 | 0.00 | 0.00 | 0.00 |
| Polyethylene glycol 400 | 0.00 | 0.00 | 0.00 | 0.00 | 0.00 | 0.00 | 0.00 | 0.00 | 0.00 |
| Phosphoric acid | 0.00 | 0.00 | 0.00 | 0.00 | 0.00 | 0.00 | 0.00 | 0.00 | 0.00 |
| Octyldodecyl myristate | 0.00 | 0.00 | 0.00 | 0.00 | 0.00 | 0.00 | 0.00 | 0.00 | 0.00 |
| Dibutylhydroxytoluene | 1.00 | 1.00 | 1.00 | 1.00 | 1.00 | 1.00 | 1.00 | 1.00 | 1.00 |
| Ascorbyl palmitate | 0.02 | 0.02 | 0.02 | 0.02 | 0.02 | 0.02 | 0.02 | 0.02 | 0.02 |
| Organic acid/mirtazapine (molar ratio) | 0.88 | 2.18 | 0.86 | 1.10 | 9.00 | 1.54 | 0.92 | 0.92 | 0.96 |

**Table 2**

| | Example 19 | Example 20 | Example 21 | Example 22 | Example 23 | Example 24 | Example 25 | Example 26 | Example 27 |
|---|---|---|---|---|---|---|---|---|---|
| Mirtazapine | 12.00 | 12.00 | 16.00 | 25.00 | 30.00 | 8.00 | 8.00 | 5.00 | 10.00 |
| Styrene-isoprene-styrene block copolymer | 32.80 | 32.80 | 29.04 | 23.10 | 20.80 | 35.37 | 24.44 | 0.00 | 0.00 |
| Polybutene | 8.20 | 8.20 | 7.26 | 5.77 | 5.19 | 8.85 | 6.11 | 0.00 | 0.00 |
| Hydrogenated rosin glycerin ester | 13.67 | 27.31 | 36.30 | 28.88 | 8.66 | 14.74 | 10.19 | 0.00 | 0.00 |
| Alicyclic saturated hydrocarbon resin | 27.31 | 13.67 | 0.00 | 0.00 | 17.33 | 29.47 | 20.37 | 0.00 | 0.00 |
| DURO-TAK87-4287 | 0.00 | 0.00 | 0.00 | 0.00 | 0.00 | 0.00 | 0.00 | 91.00 | 79.00 |
| Capric acid | 5.00 | 5.00 | 10.38 | 16.23 | 17.00 | 0.00 | 0.00 | 3.00 | 5.00 |
| Oleic acid | 0.00 | 0.00 | 0.00 | 0.00 | 0.00 | 2.55 | 0.00 | 0.00 | 0.00 |
| Caprylic acid | 0.00 | 0.00 | 0.00 | 0.00 | 0.00 | 0.00 | 0.00 | 0.00 | 0.00 |
| Isostearic acid | 0.00 | 0.00 | 0.00 | 0.00 | 0.00 | 0.00 | 0.00 | 0.00 | 0.00 |
| Stearic acid | 0.00 | 0.00 | 0.00 | 0.00 | 0.00 | 0.00 | 0.00 | 0.00 | 0.00 |
| Undecenoic acid | 0.00 | 0.00 | 0.00 | 0.00 | 0.00 | 0.00 | 0.00 | 0.00 | 0.00 |
| Lactic acid | 0.00 | 0.00 | 0.00 | 0.00 | 0.00 | 0.00 | 29.87 | 0.00 | 0.00 |
| Lauric acid | 0.00 | 0.00 | 0.00 | 0.00 | 0.00 | 0.00 | 0.00 | 0.00 | 0.00 |
| Myristic acid | 0.00 | 0.00 | 0.00 | 0.00 | 0.00 | 0.00 | 0.00 | 0.00 | 0.00 |
| Azelaic acid | 0.00 | 0.00 | 0.00 | 0.00 | 0.00 | 0.00 | 0.00 | 0.00 | 0.00 |
| Glycolic acid | 0.00 | 0.00 | 0.00 | 0.00 | 0.00 | 0.00 | 0.00 | 0.00 | 0.00 |
| Citric acid | 0.00 | 0.00 | 0.00 | 0.00 | 0.00 | 0.00 | 0.00 | 0.00 | 0.00 |
| Crotamiton | 0.00 | 0.00 | 0.00 | 0.00 | 0.00 | 0.00 | 0.00 | 0.00 | 0.00 |
| N-methyl-2-pyrrolidone | 0.00 | 0.00 | 0.00 | 0.00 | 0.00 | 0.00 | 0.00 | 0.00 | 0.00 |
| Oleyl alcohol | 0.00 | 0.00 | 0.00 | 0.00 | 0.00 | 0.00 | 0.00 | 0.00 | 0.00 |
| Sorbitan sesquioleate | 0.00 | 0.00 | 0.00 | 0.00 | 0.00 | 0.00 | 0.00 | 0.00 | 0.00 |
| Menthol | 0.00 | 0.00 | 0.00 | 0.00 | 0.00 | 0.00 | 0.00 | 0.00 | 0.00 |
| Polyethylene glycol 400 | 0.00 | 0.00 | 0.00 | 0.00 | 0.00 | 0.00 | 0.00 | 0.00 | 0.00 |
| Phosphoric acid | 0.00 | 0.00 | 0.00 | 0.00 | 0.00 | 0.00 | 0.00 | 0.00 | 0.00 |
| Octyldodecyl myristate | 0.00 | 0.00 | 0.00 | 0.00 | 0.00 | 0.00 | 0.00 | 0.00 | 5.00 |
| Dibutylhydroxytoluene | 1.00 | 1.00 | 1.00 | 1.00 | 1.00 | 1.00 | 1.00 | 1.00 | 1.00 |
| Ascorbyl palmitate | 0.02 | 0.02 | 0.02 | 0.02 | 0.02 | 0.02 | 0.02 | 0.00 | 0.00 |
| Organic acid/mirtazapine (molar ratio) | 0.64 | 0.64 | 1.00 | 1.00 | 0.87 | 0.30 | 11.00 | 0.92 | 0.77 |

| | Comparative Example 1 | Comparative Example 2 | Comparative Example 3 | Comparative Example 4 | Comparative Example 5 | Comparative Example 6 | Comparative Example 7 | Comparative Example 8 | Comparative Example 9 |
|---|---|---|---|---|---|---|---|---|---|
| Mirtazapine | 8.00 | 10.00 | 8.00 | 8.00 | 8.00 | 8.00 | 8.00 | 8.00 | 8.00 |
| Styrene-isoprene-styrene block copolymer | 36.39 | 0.00 | 34.40 | 34.40 | 34.40 | 34.40 | 34.40 | 34.40 | 34.40 |
| Polybutene | 9.10 | 0.00 | 8.60 | 8.60 | 8.60 | 8.60 | 8.60 | 8.60 | 8.60 |
| Hydrogenated rosin glycerin ester | 15.17 | 0.00 | 14.33 | 14.33 | 14.33 | 14.33 | 14.33 | 14.33 | 14.33 |
| Alicyclic saturated hydrocarbon resin | 30.32 | 0.00 | 28.65 | 28.65 | 28.65 | 28.65 | 28.65 | 28.65 | 28.65 |
| DURO-TAK87-4287 | 0.00 | 84.00 | 0.00 | 0.00 | 0.00 | 0.00 | 0.00 | 0.00 | 0.00 |
| Capric acid | 0.00 | 0.00 | 0.00 | 0.00 | 0.00 | 0.00 | 0.00 | 0.00 | 0.00 |
| Oleic acid | 0.00 | 0.00 | 0.00 | 0.00 | 0.00 | 0.00 | 0.00 | 0.00 | 0.00 |
| Caprylic acid | 0.00 | 0.00 | 0.00 | 0.00 | 0.00 | 0.00 | 0.00 | 0.00 | 0.00 |
| Isostearic acid | 0.00 | 0.00 | 0.00 | 0.00 | 0.00 | 0.00 | 0.00 | 0.00 | 0.00 |
| Stearic acid | 0.00 | 0.00 | 0.00 | 0.00 | 0.00 | 0.00 | 0.00 | 0.00 | 0.00 |
| Undecenoic acid | 0.00 | 0.00 | 0.00 | 0.00 | 0.00 | 0.00 | 0.00 | 0.00 | 0.00 |
| Lactic acid | 0.00 | 0.00 | 0.00 | 0.00 | 0.00 | 0.00 | 0.00 | 0.00 | 0.00 |
| Lauric acid | 0.00 | 0.00 | 0.00 | 0.00 | 0.00 | 0.00 | 0.00 | 0.00 | 0.00 |
| Myristic acid | 0.00 | 0.00 | 0.00 | 0.00 | 0.00 | 0.00 | 0.00 | 0.00 | 0.00 |
| Azelaic acid | 0.00 | 0.00 | 0.00 | 0.00 | 0.00 | 0.00 | 0.00 | 0.00 | 0.00 |
| Glycolic acid | 0.00 | 0.00 | 0.00 | 0.00 | 0.00 | 0.00 | 0.00 | 0.00 | 0.00 |
| Citric acid | 0.00 | 0.00 | 0.00 | 0.00 | 0.00 | 0.00 | 0.00 | 0.00 | 0.00 |
| Crotamiton | 0.00 | 0.00 | 5.00 | 0.00 | 0.00 | 0.00 | 0.00 | 0.00 | 0.00 |
| N-methyl-2-pyrrolidone | 0.00 | 0.00 | 0.00 | 5.00 | 0.00 | 0.00 | 0.00 | 0.00 | 0.00 |
| Oleyl alcohol | 0.00 | 0.00 | 0.00 | 0.00 | 5.00 | 0.00 | 0.00 | 0.00 | 0.00 |
| Sorbitan sesquioleate | 0.00 | 0.00 | 0.00 | 0.00 | 0.00 | 5.00 | 0.00 | 0.00 | 0.00 |
| Menthol | 0.00 | 0.00 | 0.00 | 0.00 | 0.00 | 0.00 | 5.00 | 0.00 | 0.00 |
| Polyethylene glycol 400 | 0.00 | 0.00 | 0.00 | 0.00 | 0.00 | 0.00 | 0.00 | 5.00 | 0.00 |
| Phosphoric acid | 0.00 | 0.00 | 0.00 | 0.00 | 0.00 | 0.00 | 0.00 | 0.00 | 5.00 |
| Octyldodecyl myristate | 0.00 | 5.00 | 0.00 | 0.00 | 0.00 | 0.00 | 0.00 | 0.00 | 0.00 |
| Dibutylhydroxytoluene | 1.00 | 1.00 | 1.00 | 1.00 | 1.00 | 1.00 | 1.00 | 1.00 | 1.00 |
| Ascorbyl palmitate | 0.02 | 0.00 | 0.02 | 0.02 | 0.02 | 0.02 | 0.02 | 0.02 | 0.02 |
| Organic acid/mirtazapine (molar ratio) | - | - | - | - | - | - | - | - | - |

### Test Example 1

The preparations obtained in Examples 1 to 18, Examples 24 to 27 and Comparative Examples 1 to 9 were observed for crystals in the following manner.

The preparation packed with an aluminum package was stored at room temperature. After storing for 1, 6 and 8 months, the preparation (10 cm²) was taken out from the aluminum package and observed for crystals of mirtazapine in the drug-containing layer with a polarization microscope. The preparation that had crystals having a particle diameter of 200 µm or less was evaluated as "pass", and the preparation that had crystals having a particle diameter exceeding 200 µm was evaluated as "fail". The results are shown in Table 3.

### Test Example 2

The preparations obtained in Examples 1 to 17 and Examples 25 to 27 were tested for cohesive force in the following manner.

The preparation packed with an aluminum package was stored at room temperature for 8 months. After storing, the preparation (10 cm²) was taken out from the aluminum package, and after peeling off the release liner of the preparation, the adhesive layer was evaluated for the cohesive force by touching with a finger. The preparation that exhibited no stringy nature and no adhesive deposit on the finger was evaluated as "pass", and the preparation that exhibited severely stringy nature and adhesive deposits on the finger was evaluated as "fail". The results are shown in Table 3.

**Table 3**

| Test specimen | Observation of crystals * | | | Property (cohesive force) ** |
|---|---|---|---|---|
| | After 1 month | After 6 months | After 8 months | |
| Example 1 | pass | pass | pass | pass |
| Example 2 | pass | pass | pass | pass |
| Example 3 | pass | pass | pass | pass |
| Example 4 | pass | pass | pass | pass |
| Example 5 | pass | pass | pass | pass |
| Example 6 | pass | pass | pass | pass |
| Example 7 | pass | pass | pass | pass |
| Example 8 | pass | pass | pass | pass |
| Example 9 | pass | pass | pass | pass |
| Example 10 | pass | pass | pass | pass |
| Example 11 | pass | pass | pass | pass |
| Example 12 | pass | pass | pass | pass |
| Example 13 | pass | pass | pass | pass |
| Example 14 | pass | pass | pass | pass |
| Example 15 | pass | pass | pass | pass |
| Example 16 | pass | pass | pass | pass |
| Example 17 | pass | pass | pass | pass |
| Example 18 | pass | pass | fail | - |
| Example 24 | pass | pass | fail | - |
| Example 25 | pass | pass | pass | fail |
| Example 26 | pass | pass | pass | pass |
| Example 27 | pass | pass | pass | pass |
| Comparative Example 1 | fail | - | - | - |
| Comparative Example 2 | pass | fail | - | - |
| Comparative Example 3 | fail | - | - | - |
| Comparative Example 4 | fail | - | - | - |
| Comparative Example 5 | fail | - | - | - |
| Comparative Example 6 | pass | fail | - | - |
| Comparative Example 7 | pass | fail | - | - |
| Comparative Example 8 | fail | - | - | - |
| Comparative Example 9 | fail | - | - | - |

| | | | | |
|---|---|---|---|---|
| Note: * pass: crystals of 200 µm or less fail: crystals larger than 200 µm -: test not performed ** pass: no adhesive deposit formed after adhering fail: adhesive deposits formed after adhering -: test not performed | | | | |

In Comparative Examples 1 and 2 that contained no solubilizer and Comparative Examples 3 to 9 that contained the other solubilizers than an organic acid, the crystalline component derived from mirtazapine (i.e., crystals larger than 200 µm) was deposited. In Examples 1 to 17 and Examples 26 and 27 that contained an organic acid, the crystalline component derived from mirtazapine (i.e., crystals larger than 200 µm) was not deposited, and good skin adhesiveness was exhibited. Among these, in Example 18 that contained no rosin derivative and Example 24 that had a content of an organic acid of 0.3 time by mole with respect to mirtazapine, the relative period of time until a crystalline component was deposited was longer than in Comparative Examples 1 to 9, and a crystalline component derived from mirtazapine was not observed until after 6 months from the production, but a crystalline component larger than 200 µm was observed after 8 months from the production. In Example 25 that had a content of an organic acid of 11.00 times by mole with respect to mirtazapine, a crystalline component derived from mirtazapine (i.e., crystals larger than 200 µm) was not deposited, but adhesive deposits of the base ingredient on the skin was observed after adhering. In Examples 1 to 17 and Examples 26 and 27 that had a content of an organic acid of from 0.58 to 9.00 times by mole with respect to mirtazapine, neither deposition of a crystalline component (i.e., crystals larger than 200 µm) nor adhesive deposits of the base ingredient were observed. The results suggested that the addition of an organic acid in the mirtazapine-containing transdermal patch of the invention was effective for preventing formation of a crystalline component derived from mirtazapine.

### Industrial Applicability

As described above, the transdermal patch of the invention is a novel patch that contains mirtazapine as the active ingredient therein and enables quantitative transdermal drug administration. The transdermal patch of the invention is free from any rapid blood level increase that is recognized with oral preparations, while exhibiting a sufficient therapeutic effect, and is therefore expected to prevent side effects. Further, the transdermal patch of the invention can be stored for a long period of time while suppressing deposition of a crystalline component derived from mirtazapine as the active ingredient with the lapse of time, and therefore enables effective and sustainable utilization of the pharmacological effect of the active ingredient therein. Consequently, the transdermal patch of the invention is a preparation effective for treatment for depression. Further, in use thereof, the transdermal patch of the invention can be differentiated from any other transdermal patches containing an antidepressant that has a different functional mechanism such as SSRI or the like, and therefore broadens the patients' latitude in selecting desired pharmaceutical preparations in treatment for depression, and is therapeutically useful.

## Claims

1. A transdermal patch comprising a support, a drug-containing layer, and a release liner, wherein the drug-containing layer contains mirtazapine and an organic acid.

2. The transdermal patch as claimed in claim 1, wherein a content of mirtazapine is of from 3 to 30% by mass relative to the drug-containing layer.

3. The transdermal patch as claimed in claim 2, wherein a content of mirtazapine is of from 5 to 25% by mass relative to the drug-containing layer.

4. The transdermal patch as claimed in any one of claims 1 to 3, wherein the organic acid is an organic acid having from 2 to 18 carbon atoms.

5. The transdermal patch as claimed in claim 4, wherein the organic acid having from 2 to 18 carbon atoms is at least one selected from the group consisting of glycolic acid, citric acid, caprylic acid, azelaic acid, capric acid, undecenoic acid, lauric acid, myristic acid, isostearic acid, stearic acid, oleic acid, and lactic acid.

6. The transdermal patch as claimed in any one of claims 1 to 5, wherein a content of the organic acid is of from 2 to 30% by mass relative to the drug-containing layer.

7. The transdermal patch as claimed in any one of claims 1 to 6, wherein a content of the organic acid is of from 3 to 24% by mass relative to the drug-containing layer.

8. The transdermal patch as claimed in any one of claims 1 to 7, wherein the organic acid is contained in an amount of from 0.3 to 11.0 times by mole the content of mirtazapine.

9. The transdermal patch as claimed in claim 8, wherein the organic acid is contained in an amount of from 0.5 to 9.0 times by mole the content of mirtazapine.

10. The transdermal patch as claimed in any one of claims 1 to 9, wherein drug-containing layer contains as a base ingredient at least one adhesive ingredient selected from the group consisting of a rubber-type adhesive base, an acrylic polymer, and a silicone polymer.

11. The transdermal patch as claimed in claim 10, wherein the adhesive ingredient is a rubber-type adhesive base and/or an acrylic polymer.

12. The transdermal patch as claimed in claim 10 or 11, wherein a content of the adhesive ingredient is of from 20 to 91% by mass relative to the drug-containing layer.

13. The transdermal patch as claimed in any one of claims 1 to 12, wherein the drug-containing layer contains as a tackifier at least one selected from a rosin derivative and an aliphatic saturated hydrocarbon resin.

14. The transdermal patch as claimed in claim 13, wherein the tackifier is a rosin derivative.

15. The transdermal patch as claimed in claim 13 or 14, wherein a content of the tackifier is of from 8 to 46% by mass relative to the drug-containing layer.

16. A method for producing a transdermal patch comprising a support, a drug-containing layer, and a release liner, the method comprising adding mirtazapine and an organic acid into the drug-containing layer.

17. A method for suppressing deposition of crystals of mirtazapine, the method comprising, in a transdermal patch comprising a support, a drug-containing layer, and a release liner, adding mirtazapine and an organic acid into the drug-containing layer.
